(19) Europäisches Patentamt

European Patent Office

Office européen des brevets

(11) **EP 1 102 615 B1**

(12) **FASCICULE DE BREVET EUROPEEN**

(45) Date de publication et mention
de la délivrance du brevet:
**27.04.2005 Bulletin 2005/17**

(51) Int Cl.⁷: **B01D 15/02**, C07C 15/08

(21) Numéro de dépôt: **00936957.0**

(86) Numéro de dépôt international:
**PCT/FR2000/001455**

(22) Date de dépôt: **26.05.2000**

(87) Numéro de publication internationale:
**WO 2000/074807 (14.12.2000 Gazette 2000/50)**

(54) **SYSTEME D'INJECTION D'UN FLUIDE DEVIE DANS UN PROCEDE DE SEPARATION EN LIT MOBILE SIMULE**

BYPASSFLÜSSIGKEITSINJEKTIONSSYSTEM IN EINEM SIMULIERTEN WANDERBETT-TRENNUNGSVERFAHREN

SYSTEM FOR INJECTING A BY-PASS FLUID IN A SEPARATION METHOD IN A SIMULATED MOVING BED

(84) Etats contractants désignés:
**ES IT**

(30) Priorité: **09.06.1999 FR 9907309**

(43) Date de publication de la demande:
**30.05.2001 Bulletin 2001/22**

(73) Titulaire: **Institut Français du Pétrole**
**92852 Rueil-Malmaison Cedex (FR)**

(72) Inventeurs:
• **HOTIER, Gérard**
**F-92500 Rueil-Malmaison (FR)**
• **FERSCHNEIDER, Gilles**
**69360 Saint Symphorien d'Ozon (FR)**
• **VIGUIE, Jean-Christophe**
**F-69360 Saint Symphorien D'Ozon (FR)**
• **DECOODT, Xavier**
**F-93100 Montreuil (FR)**

(56) Documents cités:
EP-A- 0 769 316         WO-A-95/03867
FR-A- 2 772 634         US-A- 2 985 589
US-A- 3 268 605         US-A- 3 592 612
US-A- 4 614 204

## Description

La présente invention concerne un dispositif d'injection d'un courant de fluide utilisé comme fluide dévié dans un procédé de séparation mettant en oeuvre plusieurs lits d'adsorbants, un courant de fluide principal et plusieurs fluides secondaires, les lits étant séparés par au moins un plateau distributeur de fluide, le plateau pouvant comporter un ou plusieurs panneaux distributeurs mélangeurs extracteur ou DME permettant d'injecter et/ou de mélanger et/ou d'extraire un ou plusieurs fluides secondaires par l'intermédiaire d'une seule chambre de distribution.

L'invention s'applique notamment au dispositif et au procédé décrit dans la demande de brevet du demandeur FR 2772634, lorsque l'injection et l'extraction de fluides secondaires se fait au moyen d'un seul réseau de distribution, habituellement nommé "araignée" de distribution.

L'invention concerne aussi tous les procédés où l'on cherche à améliorer la pureté d'au moins un constituant dans un mélange circulant à travers un adsorbant solide ou un catalyseur solide.

Elle peut aussi s'appliquer dans les procédés de séparation d'au moins un constituant dans un mélange pour lequel on met en oeuvre toute séparation chromatographique d'adsorption ou d'échange d'ions par exemple.

Dans la suite de la description, on désigne par les expressions "fluide principal ", un courant de fluide qui circule à travers les lits d'adsorbants, et par " fluide (s) secondaire(s) ", des fluides qui sont utilisés dans le procédé de séparation, par exemple le désorbant, la charge, l'extrait ou le raffinat et qui sont en relation avec l'extérieur.

L'invention s'applique en particulier à la séparation du paraxylène à partir de charges hydrocarbonées aromatiques à huit atomes de carbone.

L'art antérieur décrit différents dispositifs et procédés permettant d'effectuer la séparation de charges en lit mobile simulé. On peut citer les brevets US 2.985.589, US 3.214.247, US 3.268.605, US 3.592.612, US 4.614.204, US 4.378.292, US 5.200.075, US 5.316.821, et les demandes de brevets EP 0 769 316, FR 2772634 et WO 95 03867.

En règle générale, un lit mobile simulé comporte au moins trois zones chromatographiques, avantageusement quatre ou cinq, chacune de ces zones étant constituée par au moins un lit ou un tronçon de colonne.

Entre deux zones, il existe soit un point d'injection d'une charge à fractionner, soit un point d'injection d'un éluant ou désorbant, soit un point permettant de soutirer un extrait entre le point d'injection d'éluant et le point d'injection de la charge qui est situé en aval (en considérant le sens de circulation de l'éluant), soit un point de soutirage d'un raffinat entre chaque point d'injection du mélange et le point d'injection d'éluant qui est situé en aval lorsque l'on considère le sens de circulation de l'étuant.

L'ensemble des lits ou des tronçons de colonne forme une boucle fermée comprenant au moins une pompe régulée en débit permettant le recyclage du fluide principal, par exemple entre le premier et le dernier tronçon.

Au cours du procédé de séparation, on décale généralement dans un même sens, (aval ou amont, toujours en considérant le sens de circulation du fluide principal), les points d'injection et de soutirage d'au moins un tronçon ou colonne. C'est la base du principe d'un fonctionnement en lit mobile simulé.

Au cours de ce procédé, il est important que la distribution du fluide sur chacun des lits d'adsorbants se fasse de manière la plus uniforme et la plus homogène possible.

La distribution sur chacun des lits requiert une collecte du flux provenant du lit précédent (fluide principal circulant selon l'axe principal de la colonne), la possibilité d'y injecter un fluide annexe ou fluide secondaire tout en mélangeant le mieux possible ces deux fluides, ou encore la possibilité de prélever une partie du fluide collecté, de l'extraire pour l'envoyer vers l'extérieur du dispositif et aussi de redistribuer un fluide sur le lit suivant.

Pour ce faire, on peut soit faire transiter l'intégralité du fluide ou flux principal dans l'adsorbeur selon un schéma décrit dans le brevet US 2.985.589, soit faire ressortir une grande partie ou la totalité de ce flux vers l'extérieur selon un procédé décrit dans le brevet US 5.200.075.

Une autre solution consiste, comme il est décrit dans la demande de brevet FR 2772634, à faire transiter une majorité du flux principal vers l'intérieur et une minorité de ce flux vers l'extérieur, typiquement de 2 à 20 % du flux. Un des avantages d'un tel système est que les circuits d'injection et de prélèvement des fluides secondaires ont, en permanence sensiblement la même composition. Deux plateaux distributeurs sont reliés par un circuit extérieur connu sous le nom de circuit de dérivation synchrone. Ce circuit a notamment pour fonction de faire circuler la minorité de flux prélevé assurant une composition identique. Sur le circuit de dérivation, sont connectés les vannes tout ou rien de prélèvement des fluides secondaires et un clapet anti-retour. Optionnellement, le circuit peut être équipé d'une vanne tout ou rien ou encore d'une vanne de contrôle permettant d'effectuer les injections et les soutirages sur un seul plateau.

Un rinçage continu des araignées de distribution des plateaux distributeurs des unités de séparation en lit mobile simulé peut être réalisé de deux façons :

1) lorsque chacun des plateaux est équipé d'au moins deux réseaux indépendants de distribution ($D_1$ et $D_2$),on met par exemple en communication le réseau $D_1$ du plateau P avec le réseau $D_2$ du pla-

teau P+1, et le réseau $D_1$ du plateau P+1 avec le réseau $D_2$ du plateau P+2, de sorte que sur chacun des plateaux distributeurs tous les réseaux de distribution voient en permanence une circulation de fluide, et que chacun des plateaux voit s'écouler un débit de fluide dévié d'un réseau de distribution vers le fluide principal et un second débit de fluide dévié sensiblement égal depuis le fluide principal vers le second réseau de distribution. La force motrice de ces écoulements étant assurée par la perte de charge provoquée par l'écoulement du fluide principal dans le milieu granulaire poreux localisé entre deux plateaux distributeurs successifs.

2) lorsque chacun des plateaux distributeurs n'est équipé que d'un seul réseau de distribution, les circuits de dérivation ne peuvent s'établir qu'un lit sur deux, par exemple du plateau P au plateau P+1, puis du plateau P+2 au plateau P+3. En effet, si une ligne de dérivation reliait les plateaux P+1 et P+2, il en résulterait une circulation parallèle à l'adsorbeur depuis le lit de tête jusqu'au lit de fond. L'inconvénient de n'établir un circuit de dérivation qu'un lit sur deux est que les débits internes varieraient d'un lit à l'autre : sur les lits comportant un circuit de dérivation il en résulterait un débit D tandis que sur les lits ne comportant pas de circuit de dérivation il en résulterait un débit D + b.

La présente invention concerne un dispositif et un procédé particulièrement bien adaptés pour des dispositifs de séparation où les plateaux sont équipés d'un seul réseau de distribution des fluides secondaires, les panneaux distributeurs-mélangeurs-extracteurs ou DME comportant une seule chambre de distribution, extraction, et/ou mélange.

L'exigence de qualité des utilisateurs ayant évolué, le standard de pureté à atteindre est passé des valeurs données dans la fourchette (99,5 ; 99,6%) à la valeur de 99,8%. Les exploitants doivent en conséquence apporter aux unités de séparation existantes les modifications permettant d'atteindre ces nouveaux objectifs.

L'invention peut ainsi s'appliquer à des unités existantes et à des unités neuves. Elle est utilisée notamment dans les procédés et les dispositifs de séparation par adsorption dans des lits mobiles simulés à contre-courant et éventuellement dans les installations comportant une vanne tournante. Elle trouve son application par exemple dans un dispositif comportant une distribution centrale décrite par exemple dans le brevet US 4.378.292 où les plateaux distributeurs ne sont équipés que d'une seule araignée de distribution, lors d'un revamping de l'unité ou encore lors d'un changement significatif de composition de la charge qui conduit à un changement de configuration de l'unité. L'invention s'applique aussi lors du dégoulottage d'une unité.

De façon plus générale, la modification apportée par le schéma de l'invention s'applique particulièrement bien dans les cas suivants :

* dans le cas de modification de la composition de la charge liée au schéma du complexe, par exemple il est possible de changer de type d'isomérisation de manière à coproduire du benzène ou au contraire décider l'arrêt de la coproduction du benzène pour produire plus de paraxylène. Ainsi, la charge à traiter peut passer d'une teneur en éthylbenzène de 2 % et une teneur en paraxylène de 23 %, à une teneur en éthylbenzène de 15 % et une teneur en paraxylène de 17%.

* dans le cas d'opérations de dégoulottage d'une unité pour le remplacement du tamis moléculaire et/ou pour renforcer mécaniquement les plateaux distributeurs. Lorsque ce type de modification est requis, il s'accompagne d'un changement de la vanne rotative existante ; un changement de configuration nécessite de remplacer le rotor de la vanne, une augmentation de capacité de doubler la vanne rotative en plaçant une deuxième vanne en parallèle. Ces transformations se révèlent être très onéreuses et peuvent avantageusement être remplacées par la suppression de la vanne rotative et son replacement par 96 vannes tout ou rien dont le coût total est environ deux fois plus faible.

L'invention concerne un dispositif permettant de séparer au moins un composé à partir d'un mélange ou d'un corps par adsorption, en lit mobile simulé comportant au moins :

* une enceinte ou colonne, comportant un ou plusieurs lits d'adsorbants (Ai), deux lits d'adsorbants étant séparés par au moins un plateau (Pi) de distribution et d'extraction de fluides, le plateau comportant un ou plusieurs panneaux permettant de distribuer, mélanger et/ou extraire des fluides,

* au moins un conduit d'introduction d'un fluide principal et un conduit d'extraction du fluide principal,

* plusieurs conduits permettant d'extraire ou d'injecter des fluides secondaires,

* un circuit de dérivation mettant en communication un plateau de distribution avec au moins une ligne (Li,j) de dérivation,

* le panneau comporte une seule chambre (Ci) de distribution, mélange et /ou d'extraction.

Il est caractérisé en ce que :

* le dispositif comporte des moyens de mise en communication d'au moins une chambre (Ci) avec au moins une ligne (Li,j) de dérivation,

* au moins une des extrémités d'une ligne de dérivation communique avec une région Ri, R'i d'un lit d'adsorbant, ladite région étant distincte d'une chambre de distribution (Ci) et une autre extrémité est en liaison avec ladite chambre (Ci).

Les moyens de mise en communication comportent, par exemple, au moins une vanne Voi,j disposée sur au moins une ligne (Li,j) de dérivation, et l'extrémité de la ligne de dérivation qui n'est pas liée à la zone du lit d'adsorbant peut être reliée à une ligne (Ti) d'introduction et/ou d'extraction.

Les moyens de mise en communication comportent par exemple au moins une vanne rotative, ladite vanne rotative étant reliée à au moins une ligne (Ti) d'introduction et/ou d'extraction et au moins à une ligne (Li, j) de dérivation, ladite vanne comportant des moyens permettant au moins de faire communiquer une ligne d'introduction et/ou d'extraction à au moins une ligne de dérivation.

La vanne rotative permet par exemple de mettre en communication plusieurs groupes de conduits, groupe $G_1$, groupe $G_2$ et groupe $G_3$, ladite vanne comportant :

- un stator pourvu de plusieurs moyens de circulation (E, F, R, S) du ou des fluides du groupe $G_1$, des moyens de passage d'au moins deux fluides $F_1$, $F_2$ appartenant au groupe $G_3$,
- un rotor équipé de moyens de passage des fluides du groupe $G_3$ et aussi des moyens permettant la mise en communication soit des fluides de groupe $G_1$ avec le groupe $G_3$, ou bien du groupe $G_3$ avec le groupe $G_3$,
- le nombre des moyens de passage pour le fluide $F_1$ est sensiblement identique au nombre des moyens de passage pour le fluide $F_2$, ladite vanne comporte des moyens de mise en communication d'au moins deux fluides du groupe $G_3$ et en ce que la section $S_1$ de passage des lumières pour le fluide $F_1$ est différente de la section de passage $S_2$ des lumières destinées au fluide $F_2$.

Les moyens de passage de la vanne pour le fluide $F_1$ et pour le fluide $F_2$ présentent par exemple des surfaces de passage respectivement $S_1$ et $S_2$ et en ce que le rapport $S_1/S_2$ est environ égal à 4 et, de préférence, compris entre 2 et 10.

Les moyens de mise en communication des fluides de groupe $G_3$ peuvent être constitués d'encoches disposées dans une couche de matériau ou liner déposée sur la face inférieure du rotor.

Une encoche a, par exemple, une profondeur " pe " et la valeur de la profondeur est au moins égale à l'épaisseur " e " du liner.

Les moyens de circulation (E, R, S, F) disposés au niveau de la vanne, sont par exemple formés de plusieurs rainures disposées sur la face d'appui, ou face supérieure du stator et en ce que les encoches sont disposées dans le liner.

Le nombre de ces moyens de circulation est par exemple égal à 4.

La colonne peut comporter un tube central non perforé, sur au moins une partie de sa longueur, et les panneaux formant un plateau présenter une découpe de type tangentielle, la région Ri, R'i où l'on réinjecte le fluide dévié comporte au moins un moyen de distribution du fluide dévié, l'extrémité de la ligne de dérivation débouchant dans lesdits moyens, et lesdits moyens comportent une couronne annulaire montée sur le tube central.

Le circuit de distribution des fluides est par exemple disposé autour de ladite enceinte, et il peut comporter une ligne principale qui se divise en plusieurs lignes secondaires de façon que le ou les fluides atteignent les panneaux formant un plateau sensiblement au même instant.

Les plateaux peuvent avoir une découpe de type parallèle et le dispositif de distribution de fluide comporter un conduit principal, la ligne de dérivation est par exemple en liaison avec un lit d'adsorbant par l'intermédiaire d'un dispositif comportant des orifices de passage, ledit dispositif étant monté sur l'araignée de distribution de fluides.

Un plateau est par exemple délimité par une grille inférieure et une grille supérieure et l'extrémité de la ligne de dérivation en liaison avec le lit d'adsorbant est reliée à un moyen de distribution disposé au-dessus de ladite grille supérieure.

Un plateau peut être formé de plusieurs panneaux ayant une découpe de type radiale, l'enceinte comporte un tube central et une couronne de distribution des fluides secondaires associée à un plateau de distribution, des moyens de distribution du fluide dévié, lesdits moyens étant disposés en dessous de la couronne de distribution et lesdits moyens étant en liaison avec l'extrémité de la ligne de dérivation elle-même en liaison avec une région d'un lit d'adsorbant.

Les moyens de distribution du fluide dévié comportent par exemple au moins une couronne de distribution de fluide dévié, ladite couronne étant disposée à l'intérieur d'un moyen perforé (tel qu'une grille), ledit moyen présentant une forme sensiblement conique.

Le moyen perforé comporte par exemple une paroi faisant un angle $\alpha$ avec le tube central et en ce que ladite couronne est disposée à une distance a de ladite grille.

Selon un mode de réalisation, la colonne comporte un mat sensiblement central comportant un ou plusieurs éléments de mat, comportant au moins :

- une partie supérieure,
- une partie distributeur-collecteur comportant un ou plusieurs orifices secondaires et au moins un orifice principal, les sections de passage des orifices secondaires et de l'orifice principal étant différentes,
- une partie inférieure,
- la ou les parties distributeur-collecteur sont disposées entre la partie supérieure et la partie inférieure,
- un élément obturateur disposé entre la partie distributeur-collecteur et la partie inférieure,

- un élément de séparation disposé au niveau de la partie distributeur-collecteur, délimitant ainsi deux espaces de circulation de fluides.

L'invention concerne aussi un procédé d'injection d'un fluide dévié dans un procédé de séparation par lit mobile simulé comportant au moins les étapes suivantes :

\* on fait circuler un fluide principal à travers plusieurs lits d'adsorbants,

\* on injecte et on extrait des fluides secondaires (charge, désorbant, ..) selon une séquence appropriée pour réaliser la séparation des constituants à séparer de la charge,

\* on injecte un fluide dévié.

Il est caractérisé en ce que l'on fait circuler au moins une partie du fluide principal à l'extérieur de l'enceinte permettant de réaliser la séparation par l'intermédiaire d'une ligne de dérivation comportant au moins deux extrémités, une des extrémités étant en liaison avec une région d'un lit d'adsorbant distincte d'une chambre (Ci) de manière à injecter et/ou à prélever une partie du fluide principal dans la région.

On prélève par exemple à partir d'une chambre (Ci) correspondant à un plateau Pi une fraction du fluide principal et on injecte la fraction du fluide principal prélevée au niveau d'une région du lit d'adsorbant Ai+1.

On peut prélever au moins une fraction du fluide principal à partir d'une région d'un lit d'adsorbant Ai et on injecte ladite fraction prélevée dans la chambre Ci.

Le dispositif et le procédé s'applique par exemple à la séparation du paraxylène à partir de charges hydrocarbonées aromatiques à huit atomes de carbone.

L'invention sera mieux comprise au vu des figures suivantes illustrant de manière simplifiée et non limitative plusieurs modes de réalisation du dispositif et du procédé associé, parmi lesquelles :

- la figure 1 représente sur un même schéma deux variantes pour disposer des lignes de dérivation par rapport à une colonne de séparation,
- les figures 2A et 2B représentent deux variantes de réalisation utilisant des vannes de type " rotatives ",
- la figure 3 donne un exemple de dispositif d'injection du fluide dévié disposé au-dessus d'un plateau distributeur,
- la figure 4 schématise une application spécifique de l'invention à une colonne de séparation comportant une poutre ou tube central,
- les figures 5 et 6 représentent un détail du dispositif de réinjection du fluide dévié et une vue de dessous de la figure 4,
- les figures 7A, 7B, 7C représentent des plateaux présentant des panneaux ayant une découpe tangentielle,
- les figures 8, 9 et 10 schématisent différentes possibilités pour réaliser les dispositifs de distribution de fluide dévié,

- la figure 11 montre une vue de dessus d'un système de distribution disposé autour d'une colonne de séparation,
- les figures 12A, 12B, 13 et 14 représentent un exemple de réalisation d'une poutre centrale ayant une fonction de distribution de fluide,
- les figures 15 à 17 décrivent d'autres variantes de réalisation adaptées à des panneaux présentant une découpe de type tangentielle, la colonne ne comportant pas de poutre centrale, et
- les figures 18, 19 et 20 montrent une variante de réalisation d'une vanne rotative utilisée par l'exemple illustré aux figures 2A et 2B.

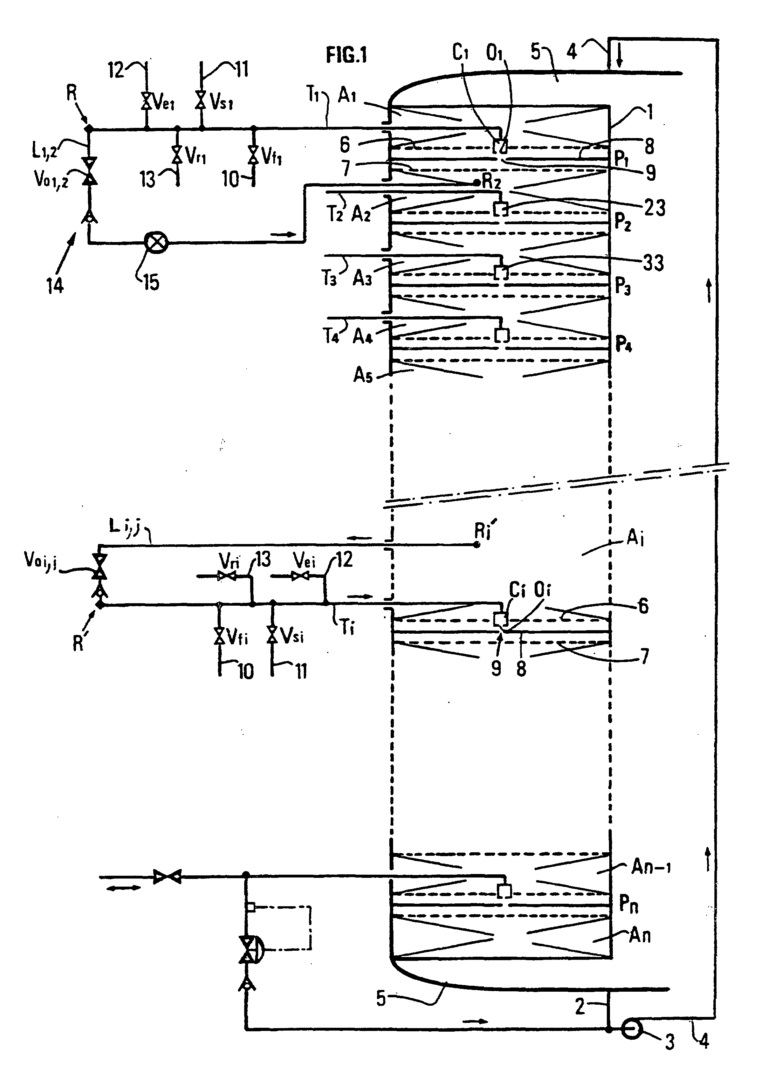

Sur la figure 1, on a représenté une colonne de séparation par chromatographie en lit mobile simulé équipée de plusieurs lignes Li,j de dérivation. Pour des raisons de simplification, cette figure schématise deux façons de procéder pour relier une ligne de dérivation à un lit d'adsorbant et à une chambre de distribution-mélange-extraction. Cette liaison sera réalisée selon l'invention entre un plateau distributeur Pi et une région d'un lit d'adsorbant disposé en amont ou en aval du plateau. Le plateau et la région du lit où l'on injecte le fluide dévié peuvent avoir le même indice ou bien des indices différents.

La colonne comporte une enceinte 1, par exemple sensiblement cylindrique, qui est remplie d'un adsorbant réparti en plusieurs lits $A_1$ à $A_n$, au moins un plateau Pi distributeur de fluides étant disposé entre deux lits d'adsorbant. Un plateau Pi comporte un ou plusieurs panneaux ou DME ayant pour fonction de distribuer, extraire et/ou mélanger un ou plusieurs fluides, chaque panneau comportant une chambre Ci de distribution-extraction-mélange qui est reliée avec l'extérieur via une araignée de distribution de fluides.

Un plateau Pi comporte notamment une grille supérieure 6 de maintien du lit d'adsorbant, une chambre Ci de distribution, extraction et/ou mélange, une grille inférieure 7, des moyens tel un baffle 8 permettant de séparer la grille inférieure 7 de la grille supérieure 6. Le baffle 8 est pourvu d'une ouverture centrale 9 permettant la circulation des fluides. La chambre Ci comporte par exemple dans sa partie inférieure un ou plusieurs orifices Oi. Ces orifices Oi laissent passer le fluide secondaire. Ce dernier est soit introduit dans le lit suivant après avoir été mélangé au fluide principal ayant traversé le lit principal, soit soutiré par la ligne de transfert appropriée.

Différentes configurations peuvent être envisagées pour les panneaux ou DME, notamment les géométries décrites dans le brevet US 5.792.346 du demandeur, pour lesquelles le panneau comporte une seule chambre de distribution, extraction et/ou mélange d'un ou plusieurs fluides.

Le fluide principal circule selon l'axe longitudinal ou axe principal de la colonne. Il est extrait par un conduit 2, recyclé par l'intermédiaire d'une pompe 3 et un conduit 4 en tête de colonne. La colonne peut être disposée selon un axe sensiblement vertical ou encore sensiblement horizontal. Le fluide principal s'écoule à l'intérieur de la colonne selon un écoulement de type piston ou plug flow, la composition et le front d'écoulement étant sensiblement uniformes en tous points de la section de la colonne. Un dispositif de distribution des fluides (non représenté sur la figure) en liaison avec le conduit 4 peut éventuellement équiper la tête de la colonne.

Une chambre Ci de distribution, extraction et/ou mélange est reliée avec l'extérieur de la colonne par l'intermédiaire d'un circuit comportant un conduit Ti relié à plusieurs lignes de transfert de fluides secondaires. Ces lignes comportent par exemple, une ligne 10 d'injection de la charge, une ligne 11 d'injection du désorbant, une ligne 12 de soutirage d'un extrait et une ligne 13 de soutirage d'un raffinat. Chaque ligne de transfert est équipée de vannes $V_{fi}$, $V_{ei}$, $V_{si}$ et $V_{ri}$ où l'indice i correspond au plateau $P_i$ et où f désigne la charge, e l'extrait, s le désorbant et r le raffinat. L'ensemble de ces vannes est relié à des moyens de permutation séquentielle adaptés à faire avancer périodiquement chaque point d'injection du fluide secondaire ou de soutirage du fluide secondaire d'un lit dans le sens de la circulation du fluide principal, c'est-à-dire du haut vers le bas, de façon à obtenir un fonctionnement en contre courant mobile simulé. Au contraire si l'on souhaite un fonctionnement en cocourant simulé, on permutera les ouvertures de vanne vers le haut dans le sens inverse de la circulation du fluide.

La colonne est pourvue dans cet exemple de réalisation d'une ligne de dérivation $L_{i,j}$ disposée par exemple entre deux plateaux $P_i$ et $P_j$, les deux plateaux pouvant être consécutifs.

Le principe de fonctionnement de ces lignes est par exemple donné dans la demande de brevet FR 2772634 dont les grandes lignes seront rappelées dans la présente demande. Une ligne de dérivation $L_{i,j}$ est pourvue d'au moins un des dispositifs mentionnés ci-après pris seul ou en combinaison, à savoir un clapet anti-retour 14, un débitmètre 15, une vanne $V_{oi,j}$ de contrôle, asservie ou non au débitmètre. Une pompe éventuellement disposée sur la ligne de dérivation peut éventuellement suppléer une insuffisance de perte de charge.

La vanne équipant le circuit de dérivation a pour référence $V_{oi,j}$ où l'indice o correspond à la fonction de dérivation et les indices i,j aux plateaux entre lesquels circule le fluide dévié.

Les mots " amont " et "aval" sont définis en considérant le sens d'écoulement du fluide principal dans la colonne.

## Première variante de réalisation

Dans la variante de réalisation donnée au niveau de la partie supérieure de la figure 1, une première extrémité de la ligne $L_{1,2}$ réalisant la dérivation du fluide entre le plateau $P_1$ et le plateau $P_2$ est reliée par un point de connexion R à la ligne $T_1$ d'introduction et/ou d'extraction de fluides qui communique avec la chambre $C_1$ et la seconde extrémité de la ligne $L_{1,2}$ est connectée par des moyens appropriés, dont certains exemples de réalisation sont détaillés ci-après, à une région référencée $R_2$ du lit d'adsorbant suivant $A_2$. Une telle disposition permet de prélever une fraction du fluide principal à partir du lit $A_1$, situé en aval du plateau $P_1$, de l'extraire par le conduit $T_1$ et de le réinjecter dans le lit d'adsorbant $A_2$ via la ligne de dérivation $L_{1,2}$ au niveau de la région référencée $R_2$. La fraction prélevée a pour fonction de balayer la ligne de dérivation et l'araignée de distribution du plateau du lit localisé $A_1$ immédiatement en aval.

Un cycle de séquence du procédé de séparation mis en oeuvre selon la première variante de réalisation peut comporter par exemple les étapes suivantes :

　　1) injection de la charge au niveau de la chambre Ci,
　　2) dévier le fluide entre Ci et Ri+1,
　　3) soutirage de l'extrait à partir de la chambre Ci,
　　4) dévier le fluide entre Ci et Ri+1,
　　5) injection du désorbant au niveau de la chambre Ci,
　　6) dévier le fluide entre Ci et Ri+1,
　　7) soutirage du raffinat à partir de la chambre Ci,
　　8) dévier le fluide entre Ci et Ri+1.

Il est bien entendu que, sans sortir du cadre de l'invention, le cycle décrit ci-dessus peut commencer à une étape différente de celle de l'injection de la charge.

## Deuxième variante de réalisation

Dans la variante de réalisation schématisée dans la partie inférieure de la figure 1, une première extrémité de la ligne de dérivation $L_{i,j}$ est connectée à un lit d'adsorbant $A_i$, par exemple au niveau d'une région $R'_i$ en utilisant des moyens appropriés, et la seconde extrémité de cette ligne est en liaison par un point R' à la ligne $T_i$ d'introduction et d'extraction de fluides secondaires. Cette variante permet de prélever à partir du lit d'adsorbant, une partie du fluide principal pour le réinjecter dans une chambre de distribution du plateau disposé en aval du point de prélèvement.

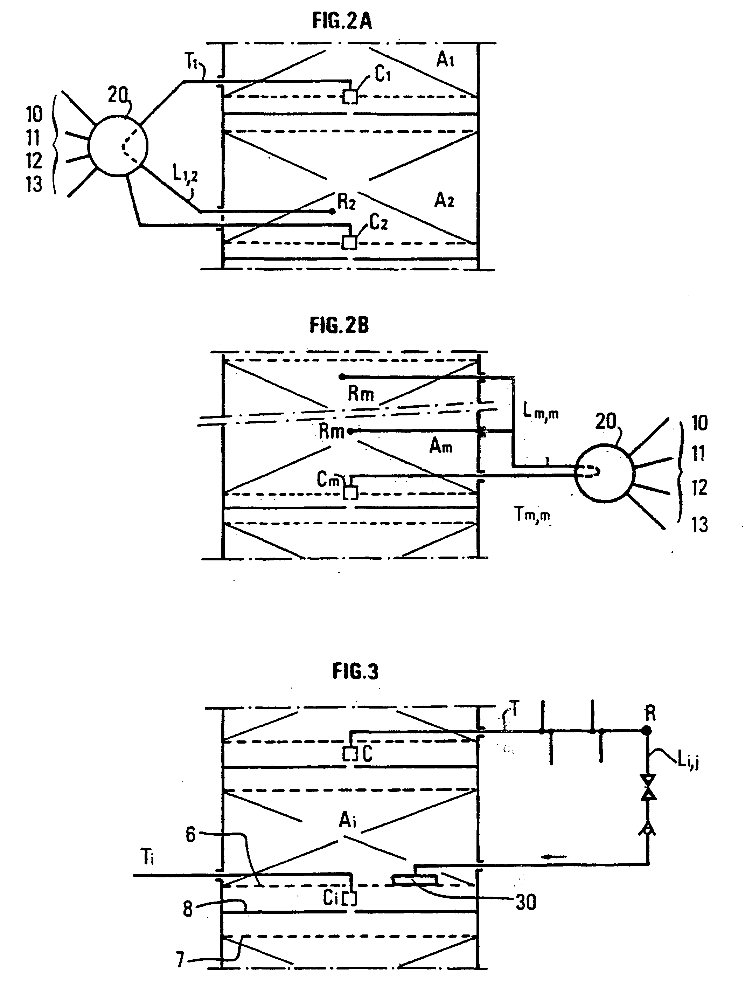

Les figures 2A et 2B schématisent une autre variante de réalisation différant de la figure 1 par le circuit d'introduction et d'extraction des fluides secondaires.

Pour ces deux variantes de réalisation, le circuit comporte une vanne rotative 20 qui a pour fonction de mettre en communication les différentes chambres Ci de distribution, extraction et soutirage avec des sources de fluide ou des conduits situés à l'extérieur de la colonne et aussi de réaliser la fonction de déviation du fluide.

Quatre lignes de transfert (10, 11, 12, 13) des fluides secondaires et identiques à celles données à la figure 1 sont reliées à la vanne rotative 20.

Au niveau de la vanne 20, on trouve plusieurs lignes de transfert Ti en liaison avec une chambre Ci, et plusieurs lignes de dérivation Li,j communiquant avec une région d'un lit d'adsorbant Ai.

La vanne rotative 20 est pourvue de moyens internes qui permettent de faire communiquer :

- soit les lignes de transfert 10, 11, 12, 13 des fluides secondaires avec les lignes de transfert Ti ,
- soit une zone $R_i$ d'un lit d'adsorbant $A_1$ avec une chambre $C_{i+1}$, d'injection, d'extraction et de mélange pour réaliser la fonction de déviation du fluide,
- soit éventuellement les lignes de transfert 10, 11, 12, 13 avec les lignes Li,j de dérivation et les lignes de transfert Ti pour réaliser la fonction de déviation ou de dérivation du fluide, et simultanément l'injection ou l'extraction.

Par exemple sur la figure 2A, pour réaliser la fonction de déviation ou de dérivation du fluide, le fluide prélevé à partir de la chambre $C_1$, circule dans la ligne $T_1$ puis à l'aide de moyens appropriés situés dans la vanne et décrits plus loin, est envoyé dans la ligne de dérivation $L_{1,2}$ au niveau de la région $R_2$ disposé dans le lit d'adsorbant $A_2$.

Sur la figure 2B, on a représenté le cas où une fraction de fluide prélevé à partir d'un lit d'adsorbant Am en un point Rm, circule à travers la ligne de dérivation Lm,m, puis par la ligne de transfert Tm,m via la vanne rotative 20 pour être introduit dans la chambre Cm.

Le principe de fonctionnement du procédé de séparation est identique à celui qui a été donné dans l'exemple de la figure 1.

Dans le cas où l'extrémité de la ligne de dérivation Li,j pour réinjecter une fraction du fluide principal dans le lit; est reliée à une zone de lit d'adsorbant selon un schéma donné dans la partie supérieure de la figure 1, les moyens de réinjection peuvent être formés par une boîte 30, disposée simplement au-dessus de la grille supérieure 6 d'un plateau Pi, permettant ainsi le passage de la fraction du fluide principal prélevé (figure 3). Les dimensions de cette boîte seront choisies, par exemple sa surface, pour que le flux de fluide par unité de surface soit respecté.

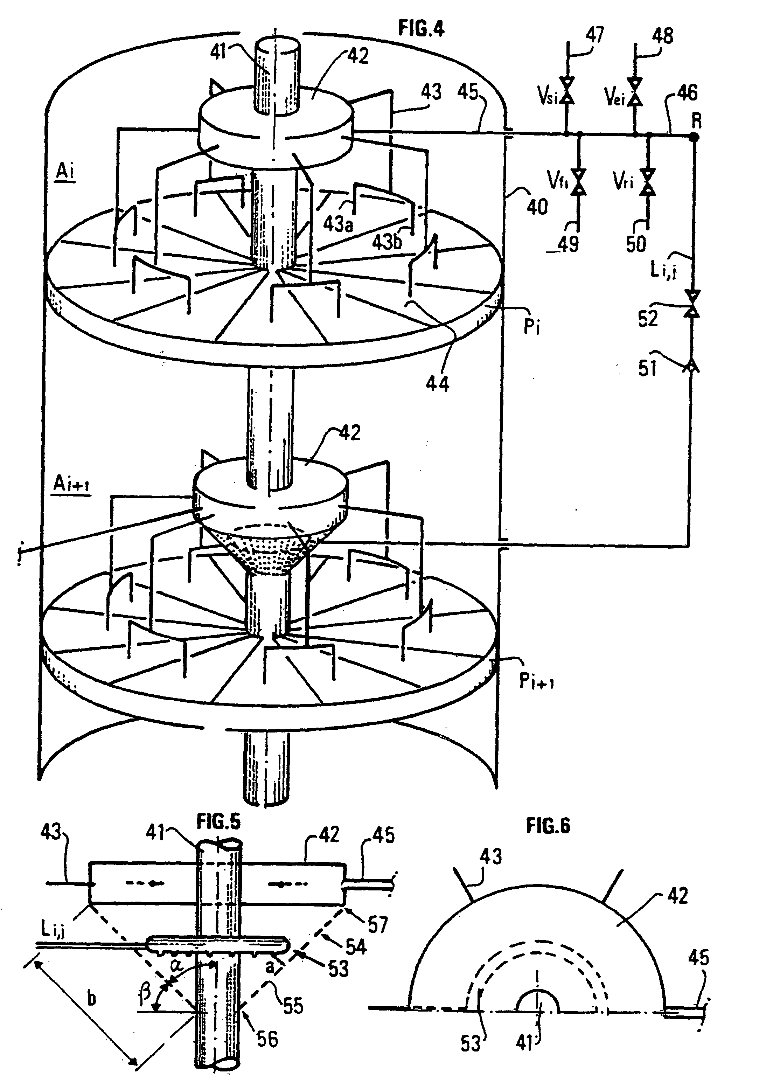

La figure 4 représente une variante de réalisation adaptée au cas où la colonne de séparation comporte une enceinte 40 comprenant un tube 41 central non perforé et aligné par exemple sensiblement selon l'axe principal de l'enceinte. Le système de distribution des fluides est constitué d'une couronne 42 par lit montée sur le tube non perforé 41. Plusieurs tuyaux 43, ou conduits de distribution de fluide, s'étendent entre un plateau distributeur Pi et la couronne de distribution de fluides associée, pour relier les différents panneaux 44 de ce plateau Pi avec l'extérieur. Dans le cas de la figure 4, chaque conduit 43 issu de la couronne de distribution 42 se subdivise en deux sous-conduits 43a et 43b qui atteigne chacun un panneau 44 et les chambres de distribution, mélange, extraction correspondantes. Un conduit de transfert 45 s'étendant entre la couronne de distribution et l'extérieur de la colonne permet la communication avec l'extérieur de la colonne et l'introduction ou l'extraction de fluides.

Selon une autre variante de réalisation, le nombre de conduits 43 peut être égal au nombre de panneaux 44.

Les panneaux 44 présentent dans cet exemple de réalisation une découpe radiale, c'est-à-dire qu'ils sont séparés par des parois radiales.

Les plateaux Pi sont disposés dans l'enceinte et maintenus à l'intérieur par des moyens appropriés et connus de l'Homme du métier. Ils peuvent être déposés sur des poutres équipant le tube central et éventuellement les parois internes de la colonne.

La ligne ou conduit 45 est raccordée à une ligne 46 sur laquelle on dispose, par exemple en Té, les lignes de transfert et les vannes reliées respectivement au désorbant 47, Vsi, à l'extrait 48, Vei, à la charge 49, Vfi et au raffinat 50, Vri.

Dans le cas où la colonne est équipée d'une ou de plusieurs lignes de dérivation Li,j, cette dernière mettant en communication deux plateaux Pi et Pi+1 par exemple, la ligne Li,j avec j=i+1, est reliée à la ligne 46 en un point R et elle est équipée par exemple d'un clapet anti-retour 51 et d'une vanne 52.

La vanne 52 est ouverte pendant toutes les périodes du cycle sauf 2 périodes particulières :

- celle durant laquelle on injecte du désorbant sur le plateau $P_1$ disposé en aval du lit $A_2$, et
- celle durant laquelle on injecte de la charge sur le lit $A_2$.

De cette manière, la charge et le désorbant sont injectés sur un seul lit à la fois. Le clapet anti-retour 51 se ferme lors du prélèvement d'extrait ou de raffinat en sortie du lit $A_1$, en supposant que la perte de charge de distribution à travers un panneau 44, un conduit 43 et la ligne 45 soit supérieure à la perte de charge à travers le lit $A_2$. En aval du clapet anti-retour 51 se trouve la ligne de dérivation Li,j qui traverse l'enceinte 40 et aboutit sous la couronne de distribution 42 pour distribuer le flux de dérivation.

Avantageusement pour réinjecter le fluide dévié, on utilise la région du lit d'adsorbant qui se trouve localisée sous la couronne de distribution 42 annulaire et pour laquelle il peut être difficile d'obtenir un chargement correct pour le tamis moléculaire. Cette région présente une zone d'écoulement fortement perturbée en l'absence de fluide dévié lors du fonctionnement de l'unité.

L'idée de la variante de réalisation donnée aux figures 4, 5 et 6, consiste donc à utiliser cette région non chargée, ou chargée de façon non homogène, pour relier l'extrémité de la ligne de dérivation qui sert à la réinjection de la fraction du fluide principal prélevé à partir d'une chambre Ci ou encore, l'extrémité de la ligne qui va servir pour prélever une partie du fluide principal pour le réinjecter au niveau d'une chambre de distribution Ci disposée en aval, selon des schémas sensiblement identiques à ceux décrits à la figure 1.

La région peut avantageusement être équipée d'un dispositif ou d'un moyen de réinjection du fluide dévié présentant une géométrie et des caractéristiques choisies en fonction de la région.

Les figures 5 et 6 présentent respectivement une vue en coupe, et de dessus d'un exemple de réalisation d'un tel moyen de réinjection de fluide, et son agencement par rapport aux éléments encombrants disposés dans la colonne, tels que la couronne annulaire et les différents conduits.

La figure 5 montre l'agencement spécifique d'une chambre annulaire, de forme sensiblement conique, disposée en dessous de la couronne de distribution et dont la fonction est notamment de répartir le fluide dévié dans le lit d'adsorbant.

Cette chambre annulaire conique est par exemple montée sur le tube central 41 non perforé. Le tube central non perforé comporte par exemple des moyens de maintien tels qu'une collerette sur laquelle viennent s'appuyer les panneaux distributeurs.

La ligne de dérivation Li,j débouche, par exemple, dans une couronne de distribution 53 (figure 5) réalisée en tube perforé. Cette couronne 53 est disposée, par exemple, à l'intérieur de la chambre annulaire de distribution délimitée par une grille 54 de type Johnson ou Nagaoka qui permet de protéger, l'extrémité de la ligne Li,j et la couronne 53 de distribution des particules d'adsorbant. Cette grille 54 peut être soudée d'une part à la périphérie inférieure de la couronne annulaire de distribution et d'autre part sur le tube central non perforé.

La grille 54 a une paroi 55, de forme sensiblement conique, qui s'appuie par exemple au niveau de son extrémité inférieure 56 sur le tube central 41 non perforé et au niveau de son extrémité supérieure 57 sous la couronne annulaire de distribution de fluide.

La géométrie de la chambre annulaire de réinjection est par exemple définie par les paramètres suivants :

$\alpha$ = angle formé par la paroi 55 et l'axe du tube central,
on peut aussi considérer l'angle $\beta$ complémentaire de l'angle $\alpha$,
b = la longueur de la paroi 55, distance qui est comprise entre les deux extrémités inférieure et supérieure 56,57.
a = la distance de positionnement de la couronne 53 de réinjection par rapport à la paroi conique 55.

La valeur de l'angle $\alpha$ correspond par exemple à l'angle de talus formé par le tamis "mal chargé " et l'axe principal de la colonne.

Les valeurs des paramètres a, b et $\alpha$ sont choisies notamment pour obtenir :

$\Rightarrow$ un montage le plus aisé possible,
$\Rightarrow$ un champ de vitesse aussi homogène que possible dans la zone du lit localisée sous la boîte annulaire de réinjection,
$\Rightarrow$ la distribution la plus homogène du liquide transitant dans la boîte annulaire de réinjection.

La distance c entre un plateau Pi et le point de liaison inférieure situé entre la grille et le tube central est choisie pour permettre un montage aisé des panneaux formant un plateau sur la collerette de support.

En utilisant ce moyen de réinjection, le débit de fluide circulant dans la ligne de dérivation permet d'une part de balayer de manière continue l'ensemble de l'araignée de distribution du lit supérieur, le conduit de transfert du lit supérieur et les lignes de transfert et de dérivation, et d'autre part d'assurer un renouvellement permanent du liquide de la boite annulaire de réinjection.

De plus le fluide dévié débouche dans une région du lit où l'écoulement serait perturbé en l'absence de dispositif de réinjection. La boîte de réinjection contribue donc à améliorer l'homogénéité de distribution de l'écoulement du fluide.

Les variantes de réalisation décrites aux figures 2A et 2B peuvent sans sortir du cadre sans sortir du cadre de l'invention, être appliqué à la colonne de séparation décrite à la figure 4, pour laquelle le moyen de réinjection des fluides dans une région "mal chargée" est détaillé à la figure 5. Les données de dimension et de géométrie spécifiées précédemment restent valables.

Sans sortir du cadre de l'invention, il est possible d'implanter un des modes de réalisation du circuit d'injection d'un fluide dévié détaillé sur les figures 1 ou 2A, 2B à une colonne utilisée pour la séparation en lit mobile simulé comportant un tube central pourvu sur certaines parties ou tronçons, de plusieurs d'orifices laissant le passage des fluides secondaires et remplaçant la couronne de distribution de type annulaire.

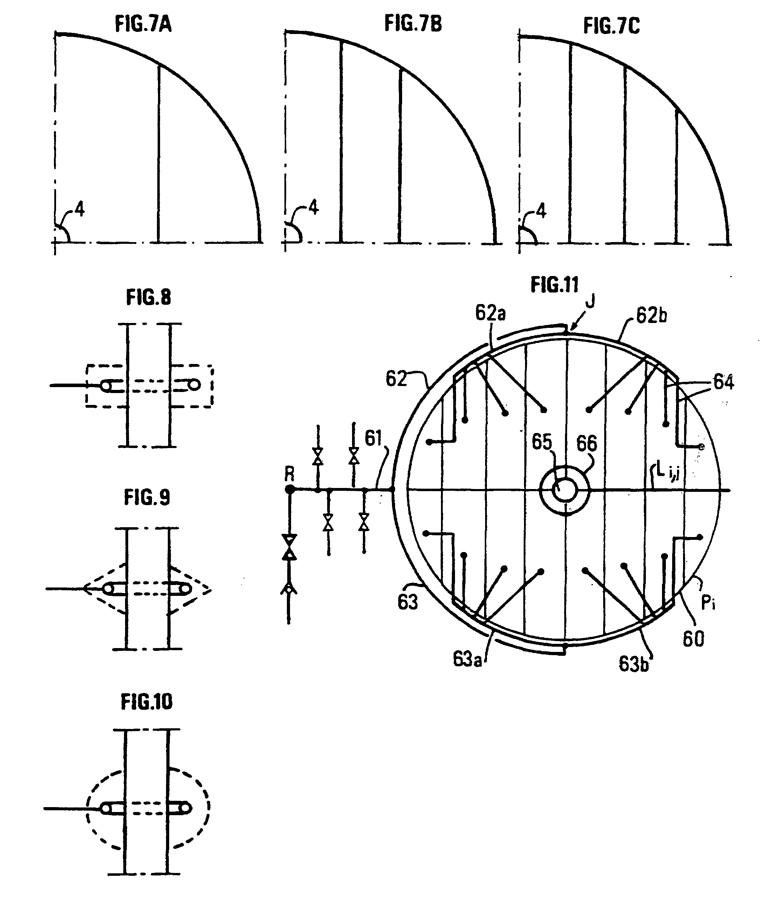

Les figures 7A, 7B, 7C schématisent plusieurs variantes de réalisation pour lesquelles le plateau Pi est formé de plusieurs panneaux ayant une découpe de type tangentiel ou parallèle.

L'araignée de distribution de fluides dans ce cas se divise, pour les exemples donnés à titre illustratif et nullement limitatif sur les figures 7A, 7B et 7C, de manière à desservir 8, 12 ou encore 16 plateaux selon des variantes illustrées par exemple dans le brevet US 5.792.346 ou encore dans les demandes de brevet FR 98/10.998 ou FR 98/10.996, en considérant le système de distribution en relation avec une seule chambre.

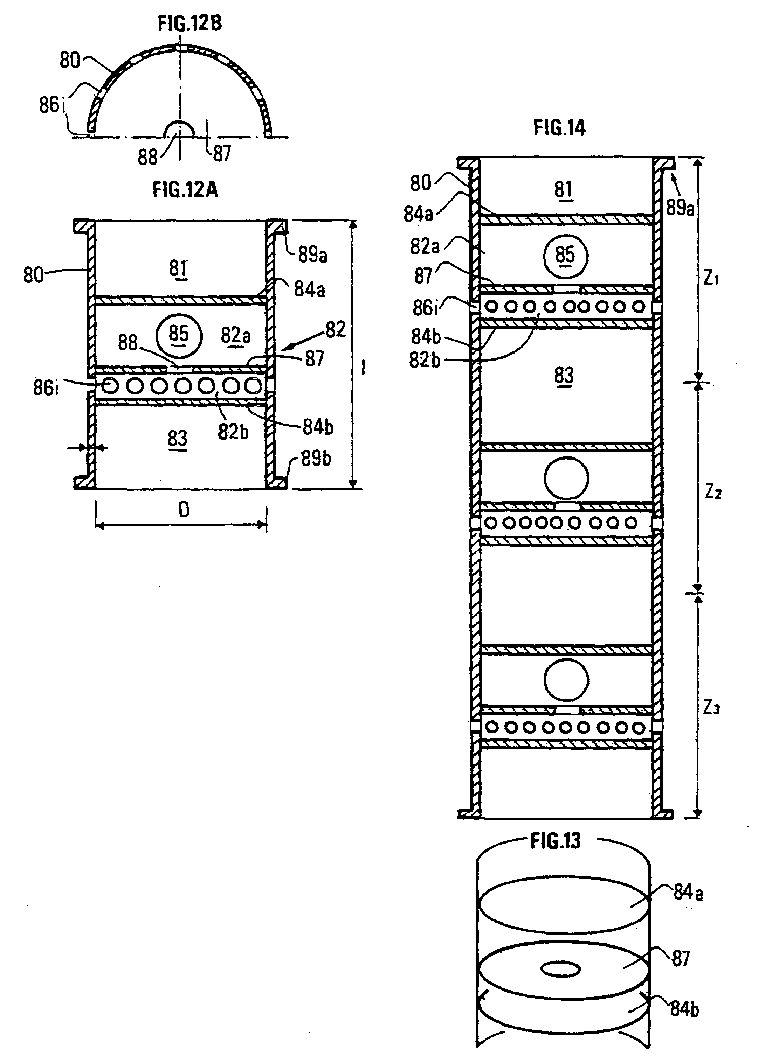

Lorsque la colonne comporte un tube central non perforé, ce dernier sert par exemple de support pour le dispositif de réinjection du fluide dévié. Le dispositif peut prendre différentes géométries dont certaines sont données à titre illustratif et nullement limitatif aux figures 8, 9 et 10.

Dans ces différentes variantes de réalisation, les parois délimitant cette chambre sont formées par une grille qui permet le passage et la redistribution du fluide dévié, et aussi d'éviter aux particules du lit d'adsorbant de passer à l'intérieur du moyen de réinjection.

La géométrie et les dimensions de ces variantes de réalisation, sont dans ce cas définies de manière à distribuer le fluide dévié de la façon la plus homogène et obtenir une répartition uniforme sur l'ensemble des différents panneaux en découpe de type tangentiel.

La figure 11 est une vue de dessus d'un système de distribution disposé à la périphérie d'une colonne de séparation 60. La colonne comporte plusieurs plateaux Pi répartis de façon sensiblement similaire à celle décrite aux figures précédentes. Chaque plateau Pi est formé de plusieurs panneaux comportant chacun une seule chambre Ci de distribution-extraction-mélange. Un plateau Pi peut être découpé en plusieurs secteurs, par exemple 4 secteurs dans cet exemple de réalisation. Chaque chambre Ci est reliée au système de distribution et d'extraction de fluides par l'intermédiaire de lignes de transferts 61 agencées par exemple de la manière donnée ci-après.

Le système de distribution comporte une ligne de transfert 61 se subdivisant en deux conduits 62, 63. Les conduits 62 et 63 se subdivisent eux-mêmes en deux sous conduits 62a, 62b et 63a, 63b permettant de distribuer et/ou extraire des fluides vers ou à partir des quatre secteurs du plateau.

L'extrémité d'un sous-conduit 62a, 62b, 63a, 63b distincte du point de jonction J comporte dans une zone donnée, un ou plusieurs conduits 64 dont le nombre est égal au nombre de panneaux formant un secteur.

La ligne de dérivation Li,j débouche dans une couronne circulaire disposée autour du tube central non perforé 65 disposée à l'intérieur d'une chambre annulaire de réinjection ou boîte annulaire référencée 66 et présentant des caractéristiques géométriques et des dimensions données précédemment aux figures 8, 9, 10.

Les figures 12A et 12B schématisent un élément d'un mat central perforé utilisé dans la colonne de séparation telle que décrite aux figures 1 et 2. Cet élément comporte un tube 80 ou une portion de tube sensiblement cylindrique ayant une épaisseur de paroi e, une longueur 1 et un diamètre interne D.

L'élément de mat est formé par exemple de trois parties, une partie supérieure 81, une partie intermédiaire 82 désignée pour la compréhension de la description " distributeur-collecteur " de fluides comportant plusieurs moyens de passage d'un ou de plusieurs fluides et une partie inférieure 83. Les parties 81 et 82, sont séparées par un élément supérieur obturateur 84a et les parties 82 et 83 par un élément inférieur obturateur 84b, afin que les fluides ne circulent que dans la partie 82. Dans certains cas une fraction minime de fluide pourra éventuellement être présente à l'intérieur des parties 81 ou 83 afin d'assurer une pressurisation de l'élément de mat, par exemple.

L'élément distributeur-collecteur est pourvu de différents moyens permettant le passage ou la circulation de fluides de l'intérieur du tronçon cylindrique vers l'extérieur et réciproquement. Ces moyens sont par exemple: un orifice 85 ayant un diamètre $\Phi_5$, plusieurs orifices 86i de diamètre $\Phi_6$, répartis sur la périphérie de l'élément de mat. Un élément de séparation 87, tel qu'une plaque pourvue par exemple d'un orifice central 88 délimite ainsi deux espaces annulaires 82a et 82b à l'intérieur de l'élément de distribution-collecte. L'espace supérieur 82a communique avec l'orifice 85 et l'espace inférieur 82b avec les orifices 86i.

Lorsque l'on considère une distribution de fluides à partir de l'élément central de la poutre, le ou les fluides à distribuer passent à travers l'orifice 85 vers l'intérieur de l'élément de mat, ensuite à travers l'orifice 88 de la plaque 87 perforée avant d'être distribués vers l'extérieur du mât à travers les orifices 86i.

Sans sortir du cadre de l'invention, il est aussi possible d'inverser les fonctions des orifices 85 et 86i, le fluide passant en premier à travers les orifices 86i puis l'orifice 85.

L'élément de mat 80 est pourvu dans sa partie inférieure et dans sa partie supérieure de brides référencées respectivement 89a et 89b, ou de tout autre moyen ayant notamment pour fonction d'assurer la liaison entre différents éléments pour former un mat utilisé comme poutre centrale.

Les éléments peuvent aussi être reliés entre eux par soudure.

L'élément de séparation 87 peut être une plaque perforée ou encore une grille.

Le diamètre de l'orifice central 88 est choisi de façon à obtenir une turbulence du ou des fluides suffisante à l'intérieur des espaces 82a et 82b pour assurer une distribution homogène des fluides au travers des orifices 86i et 85.

De préférence, l'orifice 88 est disposé au centre de la plaque 87 pour que le fluide introduit par l'orifice 85 atteigne la majorité des orifices 86i au même instant.

Lorsque l'élément de séparation 87 est constitué d'une plaque perforée, comportant plusieurs orifices, on considère la somme de la surface de passage des orifices pour assurer une distribution la plus homogène possible.

Dans certains variantes de réalisation, les parties 81 et 83 seront pressurisées de façon à obtenir sensiblement les mêmes pressions en 81, 82, 83 et éviter toute rupture mécanique au niveau des obturateurs.

Les brides 89a et 89b et l'épaisseur du mât seront conçus pour assurer la raideur suffisante par rapport à la charge à laquelle est soumise la colonne.

L'élément de mat 80 ainsi que les orifices 85 et 86i peuvent être de formes ou de géométries diverses.

La figure 14 représente un exemple d'élément de mat qui comporte, réparties sur sa longueur, trois zones $Z_1$, $Z_2$ et $Z_3$ comportant chacune une structure semblable à celle donnée à la figure 12A.

La structure de chacune des zones $Z_1$, $Z_2$ et $Z_3$ est comparable au niveau des éléments la constituant à un élément de mat comportant des moyens obturateurs 84a, 84b confinant le fluide en majorité dans la partie de distribution collecte référencée 82.

Ils comportent aussi un ou plusieurs orifices 85, des ouvertures 86i et une plaque de séparation 87 telle que décrite précédemment.

Les étapes pour fabriquer un tel élément sont par exemple les suivantes :

- on dispose d'un tube central creux sensiblement cylindrique,
- on fore les ouvertures 85 et 86i, selon des distances et avec des géométries spécifiées à l'avance,
- à l'extérieur (figure 13), on associe les différents éléments suivants :

- un élément obturateur inférieur 84b, puis juste audessus une plaque de séparation 87 et ensuite un autre élément obturateur 84a en les espaçant de façon à obtenir une structure semblable à l'intérieur de l'élément de mat décrit à la figure 12A. On recommence en disposant les mêmes éléments à partir d'une distance donnée d entre l'élément obturateur inférieur de la zone $Z_2$ et l'élément obturateur supérieur de la zone $Z_3$ par exemple,
- on glisse l'ensemble ainsi formé dans le tube creux et on le maintient à l'aide de moyens connus de l'Homme du métier. On assurera par exemple l'étanchéité entre les plaques et le tube creux par soudures ou tout autre moyen.

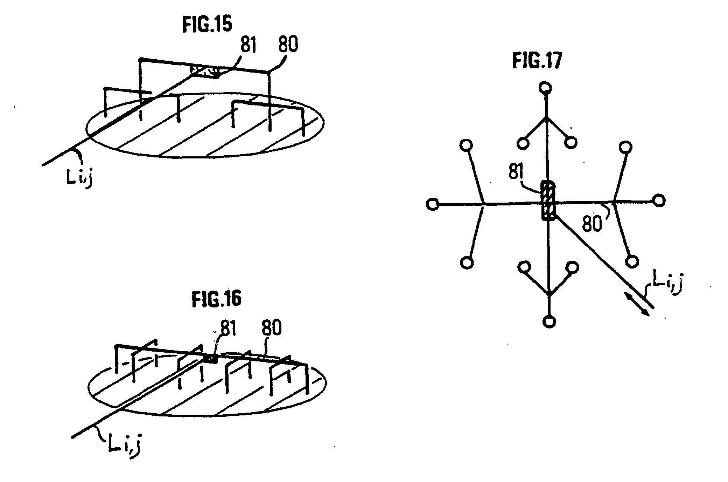

D'autres variantes de réalisation schématisées aux figures 15 à 17 sont adaptées au cas où la colonne de séparation ne comporte pas de tube central.

Ces cas sont particulièrement bien adaptés à des colonnes de séparation ayant des petits diamètres. Les plateaux Pi sont formés de panneaux à découpe tangentielle typiquement de 3 à 5.

Le dispositif ou chambre annulaire de réinjection est dans l'exemple illustré sur la figure 15 adapté de la manière suivante : la ligne de dérivation Li,j se termine par exemple par une rampe de distribution 80 montée en Té et qui est disposée à l'intérieur d'une crépine filtrante 81 préférentiellement sur l'axe principal de la colonne localisée. La ligne Li,j et/ou la crépine est par exemple solidaire de la branche centrale du râteau de distribution, si l'élévation de conduits de raccordement s'effectue dans un plan vertical.

Les figures 16 et 17 schématisent deux autres variantes de réalisation.

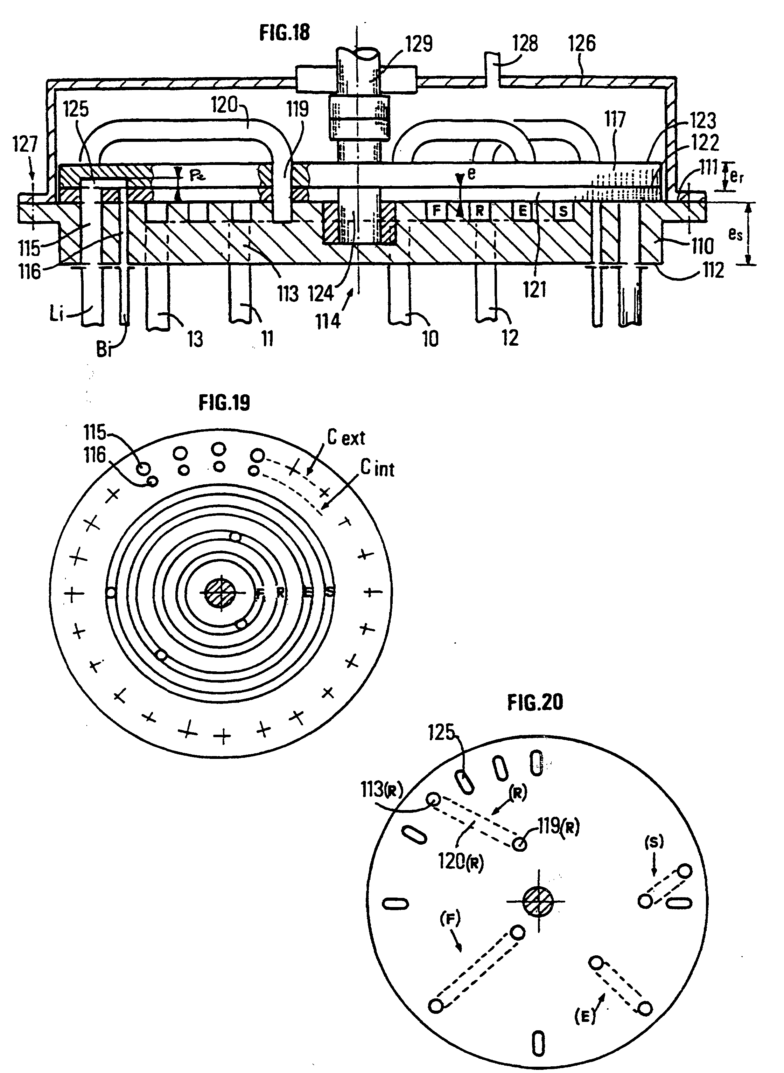

Les figures 18, 19 et 20 permettent d'illustrer la vanne selon l'invention ainsi qu'un exemple de positionnement des moyens de communication pour réaliser une étape du procédé.

Les fluides intervenant dans le procédé circulent à travers des conduits qui peuvent être classés dans trois groupes, définis par exemple selon leur fonction. La connexion entre les différents groupes est réalisée, par exemple, selon une séquence prédéterminée.

Dans un procédé de séparation mettant en jeu quatre fluides process tels que la charge F, l'extrait E, le raffinat R et le désorbant S, les différents groupes peuvent être spécifiés de la manière suivante :

- GROUPE 1, $G_1$, = les conduits permettant le transfert des fluides dits fluides process, tels que l'extrait, le raffinat, la charge, le désorbant,
- GROUPE 2, $G_2$, = les conduits permettant de réaliser la mise en communication entre les différentes lumières de la vanne rotative,
- GROUPE 3, $G_3$, = les conduits permettant la communication d'un fluide process vers un lit d'une colonne de séparation ou entre deux lits (fluide dévié).

On étendra la notion de groupe défini pour les conduits aux fluides concernés.

Les figures 18 et 19 correspondent à une vue en coupe de la vanne et une vue de dessus du stator. La vanne rotative comporte :

Un stator 110 comprenant :

♦ une pièce d'épaisseur « es », délimitée par une face supérieure 111 (face d'appui) et une face inférieure 112.

Sur la face supérieure 111 se trouvent disposées en partant du centre du stator quatre rainures F, R, E, S, sensiblement concentriques. Chacune de ces rainures est destinée au passage d'un fluide process, la distribution pouvant se faire selon un ordre F, R, E, S ou tout autre ordre. Chacune des rainures est en liaison avec un conduit 113 traversant l'épaisseur du stator et permettant le passage par exemple des fluides process qui proviennent des conduits 10, 11, 12, 13.

Dans la description, les rainures F, R, E, S font partie du groupe $G_1$ prédéfini.

Différentes possibilités existent pour faire circuler les fluides dans les rainures. Sur les figures 18 et 19, l'exemple montre une distribution des fluides qui s'effectue du plus polluant vers le moins polluant du centre 114 vers la périphérie du stator de la vanne.

♦ Plusieurs lumières :

   ♦ des lumières 115 chacune étant reliée à un conduit de transfert Li et avec une surface de passage $S_1$ sont réparties par exemple sur un cercle $C_{ext}$ (Fig 19) disposé vers la périphérie du stator. Le nombre de ces lumières 115 est égal au nombre des conduits de transfert Li,

   ♦ des lumières 116 en liaison chacune avec une ligne de dérivation (Bi) et avec une section de passage $S_2$, sont disposées sur un cercle $C_{int}$ (figure 19) situé entre le cercle le plus extérieur au stator et la première rainure du groupe (dans ce exemple la rainure F). Une lumière 116 correspond à une lumière 115,

Les sections de passage $S_1$ et $S_2$ des lumières 115 et 116 sont déterminées en fonction du débit des fluides secondaires (ou fluides process) et du débit du fluide dévié ; la perte de charge étant imposée par le milieu granulaire pour un débit donné, le diamètre de la ligne de dérivation est choisi de façon à respecter un synchronisme des débits du fluide principal et du fluide dévié. Typiquement la valeur du rapport $S_1/S_2$ est de l'ordre de 4, et peut être compris entre 2 et 10.

<u>un rotor 117</u> comprenant

♦ un élément d'épaisseur « er » délimité par une face inférieure 122 et une face supérieure 123. L'élément est monté sur un arbre de rotation comprenant deux parties 124 et 129 accouplées entre elles,
La partie 124 est maintenue au stator par des paliers. La partie 129 traverse une cloche 126 détaillée ci-après, l'étanchéité étant assurée par des systèmes connus de l'Homme du métier.

♦ plusieurs lumières 119 traversant le rotor sur son épaisseur. Ces lumières 119 sont disposées de façon à mettre en communication une rainure (R, F, S, E) avec une ligne (10, 11, 12, 13) de transfert des fluides process.

♦ des moyens 120 tels que des conduits en forme de " U " de mise en communication d'une lumière 119 avec une lumière 115 du stator. Dans ce cas d'application, les conduits 120 sont au nombre de quatre,

♦ un joint ou liner 121, d'épaisseur e, disposé sur la face inférieure 122 du rotor, assure l'étanchéité entre les quatre rainures, les différentes lumières 115, 116, 113,

♦ des moyens 125 de mise en communication d'une ligne de transfert Li avec une ligne de dérivation Bi répartis sur la face supérieure 123. Ces moyens peuvent être constitués par des encoches de forme elliptique par exemple dont les grands axes sont orientés par exemple radialement au rotor.

Les encoches 125 disposées par exemple dans le liner présentent les caractéristiques suivantes :

♦ une profondeur "Pe",

♦ un axe principal ayant une longueur suffisante pour mettre en communication deux lumières 115 et 116 situées sur un même rayon du stator afin de réaliser la déviation du fluide. La longueur de cet axe est au moins égale à la distance " d " séparant les deux cercles $C_{int}$ et $C_{ext}$.

La valeur de la profondeur "Pe" est par exemple supérieure à la valeur de l'épaisseur "e" du liner 121, l'encoche 125 étant creusée au moins en partie dans le liner disposé sur la face inférieure 122 du rotor.

<u>une cloche 126</u>

La cloche 126 est maintenue au stator à l'aide de moyens 127 connus de l'Homme du métier, tels que des vis, des boulons ou tout autre moyen permettant d'assurer une liaison mécanique. Une ligne 128 permet d'introduire un fluide sous pression. Préalablement à la rotation du rotor, la pression dans la cloche est abaissée de façon à diminuer la force s'exerçant entre le rotor et le stator et faciliter le déplacement relatif, entre ces deux pièces.

La figure 19 représente la face supérieure du stator, notamment les éléments suivants : les lumières 115 et 116 réparties selon deux cercles respectivement $C_{ext}$ et $C_{int}$, les rainures F, R, E et S et les lumières 113 débouchant dans les rainures.

La figure 20 permet d'illustrer un exemple de mise en communication des différents éléments de la vanne lors d'une étape du procédé. Sur la face inférieure du rotor, on a représenté la position des encoches et des moyens 120 lorsque quatre lits voient passer les quatre fluides process, alors que les vingt autre lits voient le fluide dévié.

Les encoches 125 permettent de laisser passer le fluide dévié entre deux lits consécutifs par exemple.

Les quatre conduits 120 en forme de " U " mettent en communication une lumière d'une rainure avec un conduit externe permettant l'introduction ou l'extraction d'un fluide process.

Ainsi sur la figure 20,

- le raffinat est extrait du lit 4, en passant à travers une lumière 119 (R), un conduit 120(R) une lumière 113 (R) et le conduit 13,
- la charge est injectée dans le lit 10, par le conduit 10, une lumière 113 (F), un conduit 120(F), une lumière 119 (F),
- l'extrait est soutiré du lit 16 en passant à travers une lumière 119 (E), un conduit 120(E) une lumière 113 ( E) et le conduit 12,
- le solvant ou désorbant est introduit dans le lit 20 par le -conduit 11, une lumière 113 (S), un conduit 120 (S), une lumière 119 (S).

Les indices R, F, S et E désignent respectivement le raffinat, la charge, le désorbant et l'extrait.

Les autres lits reçoivent le fluide dévié, ce qui correspond à mettre en communication une lumière 115 avec une lumière 116 par l'intermédiaire d'une encoche 125.

Sans sortir du cadre de l'invention il est possible de réaliser aussi une encoche elliptique dans le liner aux endroits où les fluides secondaires sont injectés ou soutirés . Dans ce cas, les fluides injectés et soutirés le sont en partie par la ligne de dérivation, mais les quatre lits qui suivent les injections ou les soutirages ne subissent pas la perturbation de débit interne due à l'interruption du courant de fluide dévié.

## Revendications

1. Dispositif permettant de séparer au moins un composé à partir d'un mélange ou d'un corps par adsorption, en lit mobile simulé comportant au moins :

   - une enceinte ou colonne, comportant un ou plusieurs lits d'adsorbants (Ai), deux lits d'adsorbants étant séparés par au moins un plateau (Pi) de distribution et d'extraction de fluides, le plateau comportant un ou plusieurs panneaux permettant de distribuer, mélanger et/ou extraire des fluides,
   - au moins un conduit (4) d'introduction d'un fluide principal et un conduit (2) d'extraction du fluide principal,
   - plusieurs conduits (10, 11, 12, 13, Ti) permettant d'extraire ou d'injecter des fluides secondaires,
   - un circuit de dérivation mettant en communication un plateau de distribution avec au moins une ligne (Li,j) de dérivation,
   - le panneau comporte une seule chambre (Ci) de distribution, mélange et /ou d'extraction,

     **caractérisé en ce que** :

   - le dispositif comporte des moyens (14, Voi,j, 20) de mise en communication d'au moins une chambre (Ci) avec au moins une ligne (Li,j) de dérivation,
- au moins une des extrémités d'une ligne de dérivation communique avec une région (Ri, R'i) d'un lit d'adsorbant, ladite région étant distincte d'une chambre de distribution (Ci) et une autre extrémité est en liaison avec ladite chambre (Ci).

2. Dispositif selon la revendication 1 **caractérisé en ce que** lesdits moyens de mise en communication comportent au moins une vanne Voi,j disposée sur au moins une ligne (Li,j) de dérivation et **en ce que** l'extrémité de la ligne de dérivation qui n'est pas liée à la zone du lit d'adsorbant est reliée à une ligne (Ti) d'introduction et/ou d'extraction.

3. Dispositif selon la revendication 1 **caractérisé en ce que** lesdits moyens de mise en communication comportent au moins une vanne rotative (20), ladite vanne rotative étant reliée à au moins une ligne (Ti) d'introduction et/ou d'extraction et au moins à une ligne (Li,j) de dérivation, ladite vanne comportant des moyens permettant au moins de faire communiquer une ligne d'introduction et/ou d'extraction à au moins une ligne de dérivation.

4. Dispositif selon la revendication 3 **caractérisé en ce que** ladite vanne rotative (20) permet de mettre en communication plusieurs groupes de conduits groupe $G_1$, groupe $G_2$ et groupe $G_3$, dans lesquels :

   - le groupe G1 concerne les conduits permettant le transfert des fluides dits fluides process, tels que l'extrait, le raffinat, la charge, le désorbant,
   - le groupe G2 concerne les conduits permettant de réaliser la mise en communication entre les différentes lumières de la vanne rotative,
   - le groupe G3 concerne les conduits permettant la communication d'un fluide process vers un lit d'une colonne de séparation ou entre deux lits,>

   ladite vanne comportant :

   - un stator (110) pourvu de plusieurs moyens de circulation (E, F, R, S) du ou des fluides du groupe $G_1$, des moyens (115, 116) de passage d'au moins deux fluides $F_1$, $F_2$ appartenant au groupe $G_3$,
   - un rotor (117) équipé de moyens (119) de passage des fluides du groupe $G_3$ et aussi des moyens (120) permettant la mise en communication soit des fluides de groupe $G_1$ avec le groupe $G_3$, ou bien du groupe $G_3$ avec le groupe $G_3$,
   - le nombre des moyens (115) de passage pour le fluide $F_1$ est sensiblement identique au nombre des moyens (116) de passage pour le fluide $F_2$, ladite vanne comporte des moyens (122) de mise en communication d'au moins deux fluides du groupe $G_3$ et **en ce que** la section $S_1$ de passage des lumières pour le fluide $F_1$ est différente de la section de passage $S_2$ des lumières destinées au fluide $F_2$.

5. Dispositif selon la revendication 4 **caractérisée en ce que** les moyens de passage de la vanne pour le fluide $F_1$ et pour le fluide $F_2$ présentent des surfaces de passage respectivement $S_1$ et $S_2$ et **en ce que** le rapport $S_1/S_2$ est environ égal à 4 et de préférence compris entre 2 et 10.

6. Dispositif selon l'une revendications 4 ou 5 **caractérisée en ce que** lesdits moyens de mise en communication des fluides de groupe $G_3$ sont constitués d'encoches (122) disposées dans une couche de matériau ou liner déposée sur la face inférieure du rotor.

7. Dispositif selon la revendication 6 **caractérisée en ce qu'**une encoche (122) a une profondeur "Pe" et **en ce que** ladite profondeur est au moins égale à l'épaisseur "e " du liner.

8. Dispositif selon l'une des revendications 6 ou 7 en ce que lesdits moyens de circulation (E, R, S, F) sont formés de plusieurs rainures disposées sur la face d'appui, ou face supérieure du stator et en ce que les encoches (122) sont disposées dans le liner.

9. Dispositif selon l'une des revendications 4 à 8 **caractérisé en ce que** les moyens de circulation (E, R, S, F) sont au nombre de 4.

10. Dispositif selon la revendication 1 **caractérisé en ce que** ladite enceinte comporte un tube central non perforé sur au moins une partie de sa longueur, et **en ce que** les panneaux formant un plateau présentent une découpe de type tangentiel, la région (Ri,R'i) comporte au moins un moyen (53, 54) de distribution du fluide dévié, l'extrémité de la ligne de dérivation (Li,j) débouche dans lesdits moyens de distribution (53, 54).

11. Dispositif selon la revendication 10 **caractérisé en ce que** le circuit de distribution des fluides est disposé autour de ladite enceinte, et **en ce qu'**il comporte une ligne principale (61) qui se divise en plusieurs lignes secondaires (62, 63, 62a, 62b, ...) afin que, le ou les fluides, atteignent les panneaux formant un plateau sensiblement au même instant.

12. Dispositif selon la revendication 1 **caractérisé en ce que** les plateaux ont une découpe de type parallèle, et **en ce que** le dispositif de distribution de fluide comporte un conduit principal, et **en ce que** la ligne de dérivation est en liaison avec un lit d'adsorbant par l'intermédiaire d'un dispositif comportant des orifices de passage, ledit dispositif étant monté sur l'araignée de distribution de fluides.

13. Dispositif selon la revendication 1 **caractérisé en ce qu'**un plateau est délimité par une grille inférieure (6) et une grille supérieure (7) et **en ce que** l'extrémité de la ligne de dérivation en liaison avec le lit d'adsorbant est reliée à un moyen de distribution (30) disposé au-dessus de ladite grille supérieure.

14. Dispositif selon la revendication 1 **caractérisé en ce qu'**un plateau est formé de plusieurs panneaux ayant une découpe de type radial, l'enceinte comporte un tube central et une couronne de distribution des fluides secondaires associée à un plateau de distribution, des moyens de distribution du fluide dévié, lesdits moyens étant disposés en dessous de la couronne de distribution et lesdits moyens étant en liaison avec l'extrémité de la ligne de dérivation elle-même en liaison avec une région d'un lit d'adsorbant.

15. Dispositif selon la revendication 14 **caractérisé en ce que** lesdits moyens comportent au moins une couronne (53) de distribution de fluide dévié, ladite couronne (53) étant disposée à l'intérieur d'un moyen perforé (55) ledit moyen présentant une forme sensiblement conique.

16. Dispositif selon la revendication 10 **caractérisé en ce que** ledit moyen perforé comporte une paroi (55) faisant un angle $\alpha$ avec le tube central et **en ce que** ladite couronne (53) est disposée à une distance a de ladite grille.

17. Dispositif selon l'une des revendications précédentes **caractérisé en ce que** ladite colonne comporte un mat sensiblement central comportant un ou plusieurs éléments de mat (80), comportant au moins :

- une partie supérieure (81),
- une partie (82) distributeur-collecteur comportant un ou plusieurs orifices secondaires (86i) et au moins un orifice principal (85), les sections de passage des orifices (85) et (86i) étant différentes,
- une partie inférieure (83),
- la ou les parties (82) distributeur-cottecteur sont disposées entre une partie supérieure (81) et une partie inférieure (83),
- un élément obturateur (84a) disposé entre la partie (82) distributeur-collecteur et la partie inférieure (83),
- un élément de séparation (87) disposé au niveau de la partie (82) distributeur-collecteur, délimitant ainsi deux espaces (82a, 82b) de circulation de fluides.

18. Procédé d'injection d'un fluide dévié dans un procédé de séparation par lit mobile simulé comportant au moins les étapes suivantes :

- on fait circuler un fluide principal à travers plusieurs lits d'adsorbants,
- on injecte et on extrait des fluides secondaires (charge, désorbant, ..) selon une séquence appropriée pour réaliser la séparation des constituants de la charge,
- on injecte un fluide dévié,

  **caractérisé en ce que** l'on fait circuler au moins une partie du fluide principal à l'extérieur de l'enceinte permettant de réaliser la séparation par l'intermédiaire d'une ligne de dérivation comportant au moins deux extrémités, une des extrémités étant en liaison avec une région d'un lit d'adsorbant distincte d'une chambre (Ci) de manière à injecter et/ou à prélever une partie du fluide principal dans la région.

19. Procédé selon la revendication 18 **caractérisé en ce que** l'on prélève à partir d'une chambre (Ci) correspondant à un plateau Pi une fraction du fluide principal et on injecte la fraction du fluide principal prélevée au niveau d'une région du lit d'adsorbant Ai+1.

20. Procédé selon la revendication 18 **caractérisé en ce que** l'on prélève une fraction du fluide principal à partir d'une région d'un lit d'adsorbant Ai et on injecte ladite fraction prélevée dans la chambre Ci.

21. Application du dispositif selon l'une des revendications 1 à 17 et du procédé selon l'une des revendications 18 à 20 à la séparation du paraxytène à partir de charges hydrocarbonées aromatiques à huit atomes de carbone.

**Patentansprüche**

1. Vorrichtung, die es ermöglicht, wenigstens eine Verbindung aus einem Gemisch oder einem Körper durch Adsorption im simulierten beweglichen Bett zu trennen, umfassend wenigstens:

- ein Gefäß oder Kolonne, umfassend eines oder mehrere Adsorptionsmittelbetten (Ai), wobei zwei Adsorptionsmittelbetten durch wenigstens einen Boden (Pi) zur Verteilung und Extraktion von Fluiden getrennt sind, wobei der Boden eines oder mehrere Felder umfasst, die es ermöglichen, Fluide zu vermischen und/oder zu extrahieren,

- wenigstens eine Leitung (4) zur Einführung eines Hauptfluids und eine Leitung (2) zum Abziehen des Hauptfluids,

- mehrere Leitungen (10, 11, 12, 13, Ti), die es ermöglichen, Nebenfluide abzuziehen oder einzuspritzen,

- eine Umleitungsleitung, die ein Verteilerboden mit wenigstens einer Umleitungsleitung (Li, j) in Kommunikation setzt,

- das Feld umfasst eine einzige Kammer (Ci) zur Verteilung, Mischen und/oder Abziehen,

  **dadurch gekennzeichnet, dass**,

- die Vorrichtung Mittel (14, Voi, j, 20) zum in Kommunikation Setzen von wenigstens einer Kammer (Ci) mit wenigstens einer Leitung (Li, j) zur Umleitung umfasst,

- wenigstens eines der Enden der Umleitungsleitung mit einer Region (Ri, R'i) eines Adsorptionsmittelbetts kommuniziert, wobei die Region unterschiedlich von der Verteilerkammer (Ci) ist und ein anderes Ende in Verbindung mit dieser Kammer (Ci) ist.

2. Vorrichtung nach Anspruch 1, **dadurch gekennzeichnet, dass** die Mittel zum in Kommunikation Setzen wenigstens ein Ventil Voi,j umfassen, angeordnet auf wenigstens einer Umleitungsleitung (Li, j), und dadurch, dass das Ende der Umleitungsleitung, das nicht mit der Adsorptionsmittelzone verbunden ist, mit einer Leitung (Ti) zur Einführung und/oder zum Abziehen verbunden ist.

3. Vorrichtung nach Anspruch 1, **dadurch gekennzeichnet, dass** die Mittel zum in Kommunikation Setzen wenigstens ein Drehventil (20) umfassen, wobei das Drehventil mit wenigstens einer Leitung (Ti) zur Einführung und/oder zum Abziehen und wenigstens einer Leitung zur Umleitung (Li,j) verbunden ist, wobei das Ventil Mittel umfasst, die es ermöglichen, eine Leitung zum Einführen und/oder zum Abziehen mit wenigstens einer Leitung zur Umleitung kommunizieren zu lassen.

4. Vorrichtung nach Anspruch 3, **dadurch gekennzeichnet, dass** das Drehventil (20) es ermöglicht, mehrere Gruppen von Leitungen Gruppe $G_1$, Gruppe $G_2$ und Gruppe $G_3$ in Kommunikation zu setzen, in denen:

- die Gruppe 1 Leitungen betrifft, die die Überführung der Fluide, die Verfahrensfluide genannt werden, wie das Extrakt, Raffinat, Beschickung und Desorptionsmittel, betreffen,

- die Gruppe 2 die Leitungen betrifft, die es ermöglichen, das in Kommunikation Setzen zwischen den verschiedenen Ausgängen des Drehventils durchzuführen, die Gruppe $G_3$ Leitungen betrifft, die die Kommunikation eines Verfahrensfluids zu einem Bett einer Trennkolonne oder zwischen zwei Betten ermöglicht,

wobei das Ventil umfasst:

- einen Stator (110), versehen mit mehreren Zirkulationsmittel (E, F, R, S) des oder der Fluide der Gruppe $G_1$, Mittel (115, 116) zum Durchlaufen wenigstens zweier Fluide $F_1$, $F_2$, die zur Gruppe $G_3$ gehören,

- einen Rotor (117), ausgerüstet mit Mitteln (119) zum Durchlaufen der Fluide der Gruppe $G_3$ und auch Mittel (120), die das in Kommunikation Setzen der Fluide von Gruppe $G_1$ mit der Gruppe $G_3$ oder auch der Gruppe $G_3$ mit der Gruppe $G_3$ ermöglichen,

- die Anzahl der Mittel (115) zum Durchlaufen für das Fluid $F_1$ ist im Wesentlichen identisch zur Anzahl der Mittel (116) zum Durchlaufen für das Fluid $F_2$, das Ventil umfasst Mittel (122) zum in Kommunikation Setzen von wenigstens zwei Fluiden der Gruppe $G_3$ und dadurch, dass der Abschnitt $S_1$ zum Durchtritt der Ausgänge für das Fluid $F_1$ verschieden vom Durchgangsquerschnitt $S_2$ der für das Fluid $F_2$ vorgesehenen Ausgänge ist.

5. Vorrichtung nach Anspruch 4, **dadurch gekennzeichnet, dass** die Durchlaufmittel des Ventils für das Fluid $F_1$ und für das Fluid $F_2$ Durchlaufoberflächen jeweils $S_1$ und $S_2$ aufweisen und dadurch, dass das Verhältnis $S_1/S_2$ etwa gleich 4 und vorzugsweise zwischen 2 und 10 ist.

6. Vorrichtung nach einem der Ansprüche 4 oder 5, **dadurch gekennzeichnet, dass** die Mittel zum in Kommunikation setzen der Fluide von Gruppe $G_3$ aus Kerben (122) bestehen, die in einer Lage aus Material oder Auskleidung bzw. Liner auf der Innenseite des Rotors angeordnet sind.

7. Vorrichtung nach Anspruch 6, **dadurch gekennzeichnet, dass** eine Kerbe (122) eine Tiefe "Pe" hat und dadurch, dass die Tiefe wenigstens gleich der Dicke "e" der Auskleidung bzw. Liner ist.

8. Vorrichtung nach einem der Ansprüche 6 oder 7, **dadurch gekennzeichnet, dass** die Mittel zur Zirkulation (E, R, S, F) gebildet sind aus mehreren Rillen, angeordnet auf der Stützseite oder oberen Seite des Stators und dass die Kerben (122) in der Auskleidung bzw. Liner angeordnet sind.

9. Vorrichtung nach einem der Ansprüche 4 bis 8, **dadurch gekennzeichnet, dass** die Zirkulationsmittel (E, R, S, F) eine Anzahl von 4 haben.

10. Vorrichtung nach Anspruch 1, **dadurch gekennzeichnet, dass** das Gefäß ein Zentralrohr umfasst, das nicht perforiert ist über wenigstens einen Teil seiner Länge und dadurch, dass die Felder einen Boden bilden, der einen Tangentialquerschnitt aufweist, wobei die Region (Ri, R'i) wenigstens ein Mittel (53, 54) zur Verteilung von umgeleitetem Fluid umfasst und das Ende der Umleitungsleitung (Li,j) in die Verteilermittel (53,54) mündet.

11. Vorrichtung nach Anspruch 10, **dadurch gekennzeichnet, dass** der Fluidverteilerkreislauf um das Gefäß herum angeordnet ist und dadurch, dass sie eine Hauptleitung (61) umfasst, die sich in mehrere Nebenleitungen (62 63, 62a, 62b, ...) unterteilt, damit das oder die Fluide die den Boden bildenden Felder im Wesentlichen zum gleichen Zeitpunkt erreichen.

12. Vorrichtung nach Anspruch 1, **dadurch gekennzeichnet, dass** die Böden einen Parallelquerschnitt haben und dadurch, dass die Vorrichtung zur Verteilung von Fluiden eine Hauptleitung umfasst und dadurch, dass die Umleitungsleitung in Verbindung mit einem Adsorptionsmittelbett über eine Vorrichtung steht, die Durchgangsöffnungen umfasst, wobei die Vorrichtung auf der Fluidverteilerspinne angebracht ist.

13. Vorrichtung nach Anspruch 1, **dadurch gekennzeichnet, dass** der Boden durch ein unteres Gitter (6) und ein oberes Gitter (7) begrenzt und dadurch, dass das Ende der Umleitungsleitung in Verbindung mit dem Adsorptionsmittelbett mit einem Verteilermittel (30), angeordnet über dem oberen Gitter, verbunden ist.

14. Vorrichtung nach Anspruch 1, **dadurch gekennzeichnet, dass** der Boden aus mehreren Feldern mit einem Radialquerschnitt gebildet ist, das Gefäß ein Zentralrohr und einen Verteilerkragen der Nebenfluide verbunden mit einem Verteilerboden umfasst, Mittel zur Verteilung umgeleiteten Fluids, wobei die Mittel unter dem Verteilerkragen angeordnet sind und die Mittel in Verbindung mit dem Ende der Umleitungsleitung selbst in Verbindung mit einem Bereich eines Adsorptionsmittelbetts sind.

**15.** Vorrichtung nach Anspruch 14, **dadurch gekennzeichnet, dass** die Mittel wenigstens einen Verteilerkragen (53) von umgeleitetem Fluid umfassen, wobei der Verteilerkragen 53 in einem perforierten Mittel (55) angeordnet ist, wobei das Mittel eine im Wesentlichen konische Form hat.

**16.** Vorrichtung nach Anspruch 10, **dadurch gekennzeichnet, dass** das perforierte Mittel eine Wand (55) umfasst, die einen Winkel $\alpha$ mit dem Zentralrohr bildet und dadurch, dass der Kragen (53) bei einem Abstand zu dem Gitter angeordnet ist.

**17.** Vorrichtung nach einem der vorhergehenden Ansprüche, **dadurch gekennzeichnet, dass** die Kolonne eine im Wesentlichen zentrale Matte umfasst, die eines oder mehrere Mattenelemente (80) umfasst, wenigstens umfassend:

- einen oberen Teil (81),

- einen Verteiler-Kollektor-Teil (82), der eine oder mehrere Nebenöffnungen (86i) und eine Hauptöffnung (85) umfasst, wobei die Durchtrittsabschnitte der Öffnungen (85) und (86i) unterschiedlich sind,

- einen unteren Bereich (83),

- der oder die Verteiler-Kollektor-Teile (82) sind zwischen einem oberen Teil (81) und einem unteren Teil (83) angeordnet,

- ein Verschließelement (84a), angeordnet zwischen dem Verteiler-Kollektor-Teil (82) und dem unteren Teil (83),

- ein Trennelement (87), angeordnet auf der Ebene des Verteiler-Kollektor-Teils (82), der zwei Räume (82a, 82b) zur Zirkulation von Fluiden begrenzt.

**18.** Verfahren zur Einspritzung eines umgeleiteten Fluids in einem Trennverfahren durch ein simuliertes bewegliches Bett, die folgenden Stufen umfassend:

- man lässt ein Hauptfluid entlang von zwei Adsorptionsmittelbetten laufen,

- man spritzt ein und man extrahiert Nebenfluide (Beschickung, Desorptionsmittel, ...) gemäß einer geeigneten Sequenz, um die Trennung der Bestandteile der Beschickung durchzuführen,

- man spritzt ein umgeleitetes Fluid ein,

**dadurch gekennzeichnet, dass** man wenigstens einen Teil des Hauptfluids nach außen von dem Gefäß zirkulieren lässt, was es ermöglicht, die Trennung mittels einer Umleitungsleitung durchzuführen, die wenigstens zwei Enden umfasst, wobei eines der Enden mit einem Bereich eines Adsorptionsmittelbetts, verschieden von einer Kammer (Ci) derart verbunden ist, dass ein Teil des Hauptfluids in den Bereich eingespritzt und/oder entnommen wird.

**19.** Verfahren nach Anspruch 18, **dadurch gekennzeichnet, dass** man aus einer Kammer (Ci), die einem Boden Pi entspricht, Hauptfluid entnimmt und man die Hauptfluidfraktion, die entnommen ist, auf dem Niveau eines Bereichs des Adsorptionsmittelbetts Ai+1 einspritzt.

**20.** Verfahren nach Anspruch 18, **dadurch gekennzeichnet, dass** man eine Hauptfluidfraktion aus einem Bereich eines Adsorptionsmittelbetts Ai entnimmt und man die entnommene Fraktion in die Kammer Ci einspritzt.

**21.** Verwendung der Vorrichtung gemäß einem der Ansprüche 1 bis 17 und des Verfahrens gemäß einem der Ansprüche 18 bis 20 bei der Trennung von para-Xylol aus aromatischen Kohlenwasserstoffbeschickungen mit 8 Kohlenstoffatomen.

**Claims**

**1.** A device allowing to separate at least one compound from a mixture or a composition by adsorption with a simulated moving bed, comprising at least :

- an enclosure or column comprising one or more adsorbent beds (Ai), two adsorbent beds being separated by at least one fluid distribution and extraction plate (Pi), the plate comprising one or more panels allowing distribution, mixing and/or extraction of the fluids,

- at least one line (4) intended for delivery of a main fluid and a line (2) intended for extraction of the main fluid,

- several lines (10, 11, 12, 13, Ti) allowing extraction or injection of secondary fluids,

- a bypass circuit communicating a distribution plate with at least one bypass line (Li,j),

- the panel comprises a single distribution, mixing and/or extraction chamber (Ci),

**characterized in that** :

- the device comprises means (14, Voi,j, 20) for communicating at least one chamber (Ci) with at least one bypass line (Li,j),

- at least one end of a bypass line communicates with a zone (Ri, R'i) of an adsorbent bed, said zone being distinct from a distribution chamber (Ci), and another end is connected to said chamber (Ci).

2. A device as claimed in claim 1, **characterized in that** said communication means comprise at least one valve Voi,j arranged on at least one bypass line (Li,j) and **in that** the end of the bypass line that is not connected to the zone of the adsorbent bed is connected to a delivery and/or extraction line (Ti).

3. A device as claimed in claim 1, **characterized in that** said communication means comprise at least one rotary valve (20), said rotary valve being connected to at least one delivery and/or extraction line (Ti) and to at least one bypass line (Li,j), said valve comprising means allowing at least to communicate a delivery and/or extraction line with at least one bypass line.

4. A device as claimed in claim 3, **characterized in that** said rotary valve (20) allows to communicate several groups of lines, group $G_1$, group $G_2$ and group $G_3$ wherein:

 - group $G_1$ concerns lines allowing to transfer fluids said "process fluids", such as extract, raffinate, feed, desorbent,
 - group $G_2$ concerns lines allowing communication between the different ports of the rotary valve,
 - group $G_3$ concerns lines allowing communication of a process fluid towards a bed of a separation columm or between two beds,

 said valve comprising :

 - a stator (110) provided with several means (E, F, R, S) intended for circulation of the fluid(s) of group $G_1^-$, means (115, 116) allowing passage of at least two fluids $F_1$, $F_2$ belonging to group $G_3$,

 - a rotor (117) equipped with means (119) allowing passage of the fluids of group $G_3$ and means (120) allowing communication of either the fluids of group $G_1$ with group $G_3$, or of group $G_3$ with group $G_3$,

 - the number of means (115) intended for passage of fluid $F_1$ is substantially equal to the number of means (116) intended for passage of fluid $F_2$, said valve comprises means (122) for communicating at least two fluids of group $G_3$ and flow section $S_1$ of the ports intended for fluid $F_1$ is different from flow section $S_2$ of the ports intended for fluid $F_2$.

5. A device as claimed in claim 4, **characterized in that** the means provided on the valve for passage of fluid $F_1$ and of fluid $F_2$ have flow surface areas $S_1$ and $S_2$ respectively and **in that** the $S_1/S_2$ ratio is about 4 and preferably ranges between 2 and 10.

6. A device as claimed in any one of claims 4 or 5, **characterized in that** said means allowing communication of the fluids of group $G_3$ consist of slots (122) provided in a layer of material or liner deposited on the lower face of the rotor.

7. A device as claimed in claim 6, **characterized in that** a slot (122) has a depth « Pe » and said depth is at least equal to the thickness « e » of the liner.

8. A device as claimed in any one of claims 6 or 7, **characterized in that** said circulation means (E, R, S, F) consist of several grooves arranged on the resting face or upper face of the stator and **in that** slots (122) are provided in the liner.

9. A device as claimed in any one of claims 4 to 8, **characterized in that** circulation means (E, R, S, F) are 4 in number.

10. A device as claimed in claim 1, **characterized in that** said enclosure comprises a non-perforated central tube over at least part of the length thereof, and **in that** the panels forming a plate exhibit a tangential type cutout, zone (Ri, R'i) comprises at least one diverted fluid distribution means (53, 54), the end of bypass line (Li,j) opens into said distribution means (53, 54).

11. A device as claimed in claim 10, **characterized in that** the fluid distribution circuit is arranged around said enclosure and **in that** it comprises a main line (61) divided into several secondary lines (62, 63, 62a, 62b, ...) so that the fluid(s) reach the panels forming a plate substantially at the same time.

12. A device as claimed in claim 1, **characterized in that** the plates exhibit a parallel type cutout and **in that** the fluid distribution device comprises a main line, the bypass line is connected to an adsorbent bed by means of a device comprising transfer ports, said device being mounted on the fluid distribution spider.

**13.** A device as claimed in claim 1, **characterized in that** a plate is delimited by a lower grid (6) and an upper grid (7) and **in that** the end of the bypass line connected to the adsorbent bed is connected to a distribution means (30) arranged above said upper grid.

**14.** A device as claimed in claim 1, **characterized in that** a plate consists of several panels exhibiting a radial type cutout, the enclosure comprises a central tube and a secondary fluid distribution ring associated with a distribution plate, diverted fluid distribution means, said means being arranged below the distribution ring and said means being connected to the end of the bypass line, itself connected to a zone of an adsorbent bed.

**15.** A device as claimed in claim 14, **characterized in that** said means comprise at least one diverted fluid distribution ring (53), said ring (53) being arranged in a perforated means (55), said means having a substantially conical shape.

**16.** A device as claimed in claim 10, **characterized in that** said perforated means comprises a wall (55) forming an angle $\alpha$ with the central tube and **in that** said ring (53) is located at a distance a from said grid.

**17.** A device as claimed in any one of the previous claims, **characterized in that** said column comprises a substantially central mast comprising one or more mast elements (80), including at least :

- an upper part (81),

- a distributor-collector part (82) comprising one or more secondary ports (86i) and at least one main port (85), the flow sections of ports (85) and (86i) being different,

- a lower part (83),

- distributor-collector part(s) (82) are arranged between an upper part (81) and a lower part (83),

- a sealing element (84a) arranged between distributor-collector part (82) and lower part (83),

- a separation element (87) arranged on distributor-collector part (82), thus delimiting two fluid circulation spaces (82a, 82b).

**18.** A process intended for injection of a diverted fluid in a simulated moving bed separation process, comprising at least the following stages :

- circulating a main fluid through several adsorbent beds,

- injecting and extracting secondary fluids (feed, desorbent, ...) according to a suitable sequence in order to achieve separation of the constituents of the feed,

- injecting a diverted fluid,

**characterized in that** at least part of the main fluid is circulated outside the enclosure allowing separation by means of a bypass line comprising at least two ends, one end being connected to a zone of an adsorbent bed distinct from a chamber (Ci) so as to inject and/or to extract part of the main fluid in the zone.

**19.** A process as claimed in claim 18, **characterized in that** a fraction of the main fluid is drawn off from a chamber (Ci) corresponding to a plate Pi and the main fluid fraction drawn off is injected into a zone of adsorbent bed Ai+1.

**20.** A process as claimed in claim 18, **characterized in that** a fraction of the main fluid is drawn off from a zone of an adsorbent bed Ai and said fraction is injected into chamber Ci.

**21.** Application of the device as claimed in any one of claims 1 to 17 and of the process as claimed in any one of claims 18 to 20 for separation of paraxylene from aromatic hydrocarbon-containing feeds with eight carbon atoms.

**FIG.1**

**FIG.2A**

**FIG.2B**

**FIG.3**

FIG.4

FIG.5

FIG.6

**FIG.7A**

**FIG.7B**

**FIG.7C**

**FIG.8**

**FIG.11**

**FIG.9**

**FIG.10**

**FIG.12B**

80

86i

88    87

**FIG.12A**

89a

80

81

84a

88    85    82a    82

87

86i

82b    84b

83

89b

D

**FIG.14**

80

84a

82a

87

86i

84b

82b

81

85

89a

$Z_1$

83

$Z_2$

$Z_3$

**FIG.13**

84a

87

84b

**FIG.15**

81    80

L i,j

**FIG.17**

81

80

L i,j

**FIG.16**

81    80

L i,j

**FIG.18**

**FIG.19**

**FIG.20**